# EUROPEAN PATENT APPLICATION

(11) **EP 4 129 309 A1**
(43) Date of publication of application: **08.02.2023**
(21) Application number: 20927889.4
(22) Date of filing: 13.11.2020
(51) Int. Cl.: A61K 35/16, A61K 39/42, A61K 39/40, A61P 31/14, A61P 31/04, A61P 31/12, A61K 35/12

(54) **METHOD FOR PRODUCING SERUM COMPOSITION FOR PREVENTING OR TREATING MUCOSA-RELATED INFECTIOUS DISEASE IN YOUNG MAMMALS, SERUM COMPOSITION PRODUCED THEREBY, AND USE THEREOF**

(30) Priority: 24.03.2020 KR 20200035466; 28.08.2020 KR 20200109320
(71) Applicant: Ahn, Byung Chul, Daegu 42273 (KR)
(72) Inventor: Ahn, Byung Chul, Daegu 42273 (KR)
(74) Representative: M. Zardi & Co S.A.
(86) International application number: PCT/KR2020/015986
(87) International publication number: WO 2021/194038

(57) **Abstract**

The present invention provides a method of producing a serum containing protective antibodies with mucosal immunity, a PAMI serum, against mucosa-related infectious pathogens from an adult pig or cattle comprising a step of administering the mucosa-related infectious pathogens to the adult pig or cattle via a mucosal route such as oral or nasal administration; and a method of preventing and/or treating mucosal infectious diseases in piglets and calves by orally or intravenously administering the PAMI serum produced by the method above to piglets or calves. In experiments conducted by the method of the present invention, piglets and young calves were successfully prevented and/or treated from infection with mucosa-related infectious pathogens.

## Description

### FIELD OF THE INVENTION

The present invention relates to a method of preventing and/or treating mucosa-related infectious diseases in young mammals. In more detail, the present invention provides a method of producing a serum containing protective antibodies with mucosal immunity (PAMI serum) against infectious pathogens from an adult mammal by administering the mucosa-related infectious pathogens via an oral or nasal route and a method of preventing and/or treating mucosa-related infectious diseases of young mammals by orally or intravenously administering the PAMI serum into a neonatal or young mammal.

### BACKGROUND OF THE INVENTION

Mucosa-related infectious diseases such as digestive, respiratory, and genital infectious diseases in pigs, horses, and ruminants cause mortality and decrease growth performance in young mammals, and accordingly, it has led to tremendous economic losses in the livestock industry worldwide.

One of the effective ways to prevent infectious diseases for young mammals is to acquire protective antibodies against infectious pathogens from the mother. Such passive immunity from the mother is the main early defense means against infectious pathogens until the offspring become immunologically mature.

Therefore, as an effective way of inducing humoral immunity against infectious pathogens from the pregnant mother and then transferring protective antibodies to the offspring through the placenta during pregnancy or colostrum after birth, maternal vaccination has been used to protect young mammals from infectious diseases.

For example, in nonmucosa-related infectious pathogens such as Japanese B encephalitis virus and classical swine fever virus, vaccination of pregnant sows via intramuscular or subcutaneous route leads to induction of the humoral immunity against the infectious pathogens, and the maternal colostrum-fed piglets are effectively protected from the infection with the nonmucosa-related infectious pathogens (van Oirschot JT. 2003. Vet Microbiol 96:367-384; Fan YC et al., 2013. Vet Microbiol 163:248-256).

However, vaccines are not always available for all infectious pathogens. Especially, in mucosa-related infectious pathogens such as enteric, respiratory, or reproductive infectious pathogens, the vaccination via the intramuscular or subcutaneous route has limited effectiveness in preventing the mucosa-related infectious disease of young mammals. For example, in mucosa-related infectious pathogens such as enterotoxigenic E. coli (ETEC), transmissible gastroenteritis virus (TGEV), porcine epidemic diarrhea virus (PEDV), group A porcine rotavirus (GAPRV), bovine coronavirus (BCoV), and bovine group A rotavirus (BGARV), the serum from pregnant mothers intramuscularly vaccinated with these infectious pathogens shows high viral neutralization (VN) titer to the viral pathogens and high agglutination (AG) titer to the bacterial pathogens. However, the vaccinated maternal colostrum-fed offsprings are not effectively protected from these mucosa-related infectious pathogens (Mostl and Burki, 1988, 35:186-196. J. Vet. Med. B; Lin et al., 2019, 15:26-37, BMC Veterinary Research).

As alternative ways to overcome these obstacles, egg yolk-originated immunoglobulin Y (IgY) and cow colostrum antibodies have been used to prevent mucosa-related infectious diseases in piglets, and hyperimmune serums have been used to prevent mucosa-related infectious diseases in calves. However, effectiveness has remained unsatisfactory.

Considering the lack of effective ways to control mucosa-related infectious pathogens, the widespread prevalence of infectious diseases with high morbidity and mortality, and the global population of approximately 1 billion pigs and 1.5 billion cattle (data source: Food and Agriculture Organization of the United Nations), mucosa-related infectious diseases of the piglets and calves have been the cause of the tremendous economic loss in the livestock industry worldwide. Thus, there is an urgent need to develop more effective methods for preventing and/or treating infection with mucosa-related infectious pathogens, such as digestive, respiratory, or genital track infectious pathogens, in piglets and calves.

Meanwhile, Russian Pat. No. RU2438709C1 discloses "Serum against cattle diseases caused by viruses of infectious rhinotracheitis, paraflu, rota, corona and mucosa diarrhea-disease, polyspecific, hyperimmune, method of prevention and/or treatment of cattle diseases caused by viruses of infectious rhinotracheitis, parainfluenza, rota, corona and mucosa diarrhea-disease", and Russian Pat. No. RU2396979C2 discloses "Hyperimmune polyvalent serum against mass virus disease of cattle". However, the inventions described above do not mention a serum containing protective antibodies with mucosal immunity capable of protecting against mucosa-related infectious pathogens and a method of preparing a serum composition containing protective antibodies with mucosal immunity. In addition, it was confirmed in the test result that a hyperimmune serum of an adult cattle immunized with an infectious pathogen or a part of a pathogen via an intramuscular or subcutaneous route failed to protect a calf from the mucosa-related infectious pathogens (Selim et al., 1995. Vaccine 13:1454-1459).

### DETAILED DESCRITION OF THE INVENTION

### TECHNICAL PROBLEMS

The objects of the present invention derived from the above needs are to provide a serum containing protective antibodies with mucosal immunity against mucosa-related infectious pathogens, named a PAMI serum, to provide a method of inducing and producing the PAMI serum, and to provide a method of preventing and/or treating mucosa-related infectious diseases in young mammals.

### SUMMARY OF THE INVENTION

As the ways of achieving objects described above, the present invention provides a method of producing a serum containing protective antibodies with mucosal immunity capable of protecting against infection with the mucosa-related infectious pathogens, from an adult mammal, by administering the mucosa-related infectious pathogens of young mammals to an adult mammal via a mucosal route such as an oral or nasal route.

The present invention provides a serum containing the protective antibodies with mucosal immunity capable of protecting against infection with the mucosa-related infectious pathogens, and more particularly, a serum containing secretory immunoglobulin A (slgA), being secreted to a mucosa membrane as a primary antibody, capable of protecting young mammals from mucosa-related infectious diseases.

The present invention provides a method of preventing and/or treating the mucosa-related infectious diseases in young mammals by orally or intravenously administering an effective amount of a serum induced and produced by the method above into neonatal or young mammals at an optimal time.

### TECHNICAL EFFECTS OF THE INVENTION

Considering the population of domestic and international mammals, and the morbidity, mortality, and decreased growth performance of young mammals infected with mucosa-related infectious pathogens, the method of the present invention capable of preventing and/or treating mucosa-related infectious diseases of piglets and calves will affect a tremendous economic impact on the livestock industry worldwide.

According to the present invention, the PAMI serum containing various and multiple protective antibodies with mucosal immunity against multiple mucosa-related infectious pathogens can be consistently induced, maintained, and produced from one individual mammal by administering multiple mucosa-related infectious pathogens to the individual mammal.

The method of preventing and/or treating infection with various mucosa-related infectious pathogens in piglets and calves infected with different mucosa-related infectious pathogens can be applied to mucosa-related infectious pathogens such as the TGEV, the PEDV, the GAPRV, the BCoV, the BGARV, and the E. coli as well as to all mucosa-related infectious viral and bacterial pathogens that cause digestive, respiratory, or genital infectious diseases in the piglets and calves, and can be used to prevent and/or treat infection with these mucosa-related infectious pathogens.

The method of the present invention according to another embodiment can adjust a protection period of PAMI serum-administered piglets or calves from infectious pathogens by properly adjusting the quantity and titers of the PAMI serum.

The method of the present invention of preventing and/or treating infection with various mucosa-related infectious pathogens in the two different species of mammals can be applied not only to the two different mammals including pigs and cattle but also to all mammals including horses, ruminants, and humans with a similar immune system for preventing and/or treating infection with mucosa-related infectious pathogens in young mammals.

According to the present invention, early separation of PAMI serum-fed piglets from the sow can solve the problems such as piglet-crushing by sows and insufficient milk supply of sows and then this can lead to an increase in the number of piglets per sow per year (PSY).

The method of the present invention can apply to all mammals with a similar immune system and replace existing vaccines in protecting young mammals from infectious pathogens and can be used as a way of preventing and/or treating infections with mucosa-related infectious pathogens that the existing vaccines cannot solve.

### BRIEF DESCRIPTION OF THE DRAWINGS

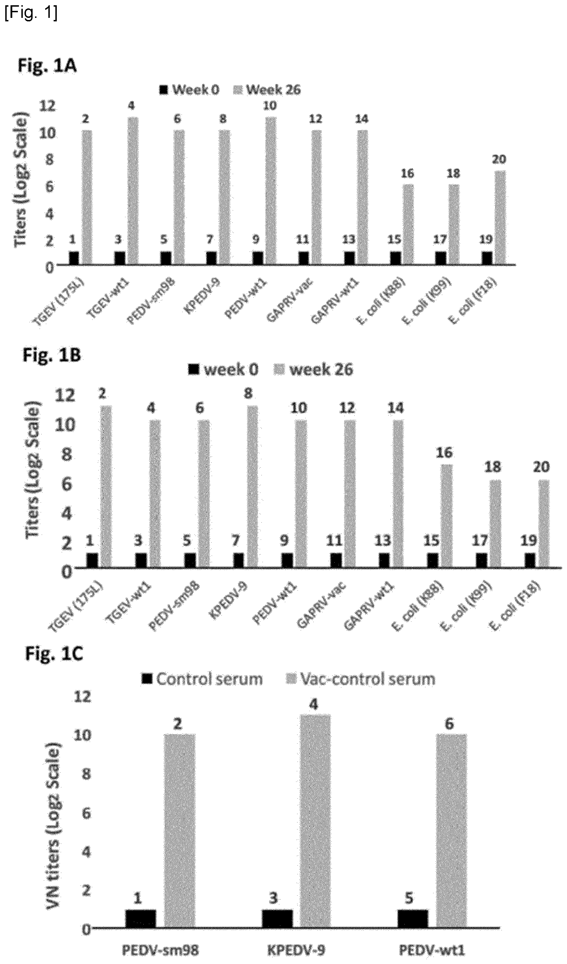

FIGs. 1A to 1C show a viral neutralization (VN) titer and an agglutination (AG) titer of serums of adult pigs. The adult pigs were infected with infectious pathogens such as the TGEV (175L), the TGEV-wt1, the PEDV-m98, the KPEDV-9, the PEDV-wt1, the GAPRV-vac, the GAPRV-wt1, and the E. coli (K88, K99 and F18), every week for 26 weeks.
   For the test, a control serum and a PAMI serum were collected from the adult pig mock-infected with infectious pathogens (week 0) and the adult pig infected with infectious pathogens (week 26), respectively.
   In addition, from the adult pig intramuscularly vaccinated with the PEDV-sm98 or the KPEDV-9, a control serum before vaccination and a vac-control serum after vaccination were collected, respectively. The VN titers of the serums against the TGEV-wt1, the PEDV-sm98, the KPEDV-9, PEDV-wt1, the GAPRV-vac and the GAPRV-wt1, and the AG titers of the serums against the E. coli (K88, K99, F18) were examined according to the materials and methods, respectively. The number on each graph means a corresponding lane or column.
FIG. 1A shows the VN titers against the TGEV (175L), the TGEV-wt1, the PEDV-m98, the KPEDV-9, the PEDV-wt1, the GAPRV-vac, and the GAPRV-wt1, and the AG titers of the E. coli (K88, K99 and F18) in a control serum and a PAMI serum collected from castrated male pigs before and after oral infection with the TGEV (175L), the TGEV-wt1, the PEDV-m98, the KPEDV-9, the PEDV-wt1, the GAPRV-vac, the GAPRV-wt1, and the E. coli (K88, K99 and F18), respectively.
FIG. 1B shows the VN titers against the TGEV (175L), the TGEV-wt1, the PEDV-m98, the KPEDV-9, the PEDV-wt1, the GAPRV-vac, and the GAPRV-wt1, and the AG titers against the E. coli (K88, K99 and F18) in a control serum and a PAMI serum collected from female pigs before and after oral infection with the TGEV (175L), the TGEV-wt1, the PEDV-m98, the KPEDV-9, the PEDV-wt1, the GAPRV-vac, the GAPRV-wt1, and the E. coli (K88, K99 and F18), respectively.
FIG. 1C shows the VN titers of a control serum and a vac-control serum against the PEDV-sm98, the KPEDV-9, and the PEDV-wt1, respectively.

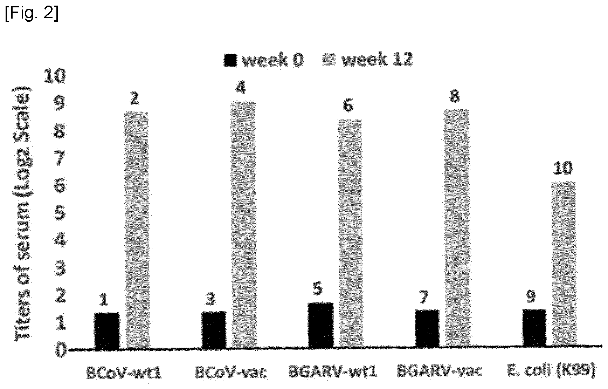

FIG. 2 shows viral neutralization (VN) and bacterial agglutination (AG) titers of a serum of an adult cow. The adult cow was weekly infected with the BCoV-wt1, the BCoV-vac, the BGARV-wt1, the BGARV-vac, and the E. coli (K99) via an oral route for 12 weeks. The serums before (week 0) and after (12 weeks) infection with the pathogens were collected and then named the control serum and the PAMI serum, respectively. The VN titers of the control serum and the PAMI serum against the BCoV-wt1, the BCoV-vac, the BGARV-wt1, and the BGARV-vac and the AG titers of the control serum and the PAMI serum against the E. coli(K99) were examined according to materials and methods, respectively. The number on each graph means a corresponding lane or column.

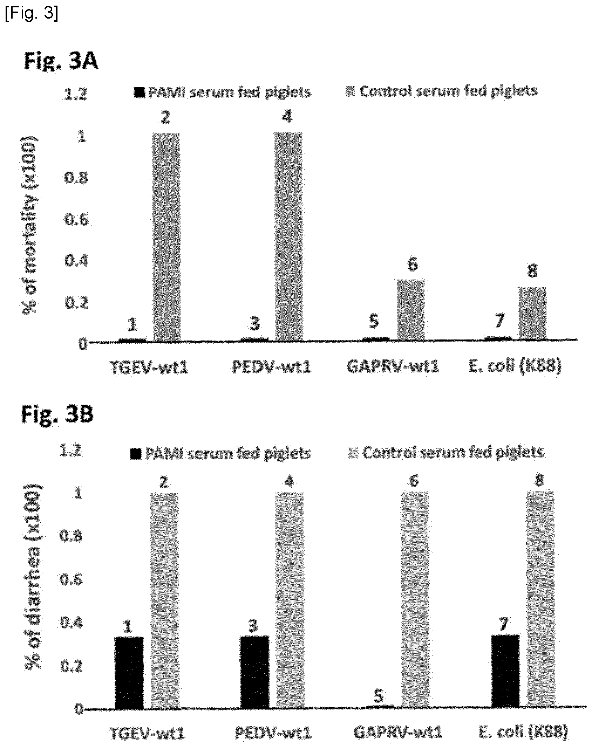

FIGs. 3A and 3B show percentages of mortality and diarrhea incidence in the control serum or PAMI serum-fed piglets, respectively. The control serum and PAMI serum were orally administered to a control serum-fed piglets' group and a PAMI serum-fed piglets' group, respectively. Then, the piglets' group A, the piglets' group B, the piglets' group C, and the piglets' group D were challenged with the TGEV-wt1, the PEDV-wt1, the GAPRV-wt1, and the E. coli (K88), respectively, and clinical outcomes of the piglets were examined according to the materials and methods described herein. The number on each graph means a corresponding lane or column.
FIG. 3A shows percentages of mortality of the piglets' group A challenged with the TGEV-wt1, the piglets' group B challenged with the PEDV-wt1, the piglets' group C challenged with the GAPRV-wt1, and the piglets' group D challenged with the E. coli (K88), respectively. The number on each graph means a corresponding lane or column.
FIG. 3B shows percentages of diarrhea incidence of the piglets' group A challenged with the TGEV-wt1, the piglets' group B challenged with the PEDV-wt1, the piglets' group C challenged with the GAPRV-wt1 and the piglets' group D challenged with the E. coli (K88), respectively. The number on each graph means a corresponding lane or column.

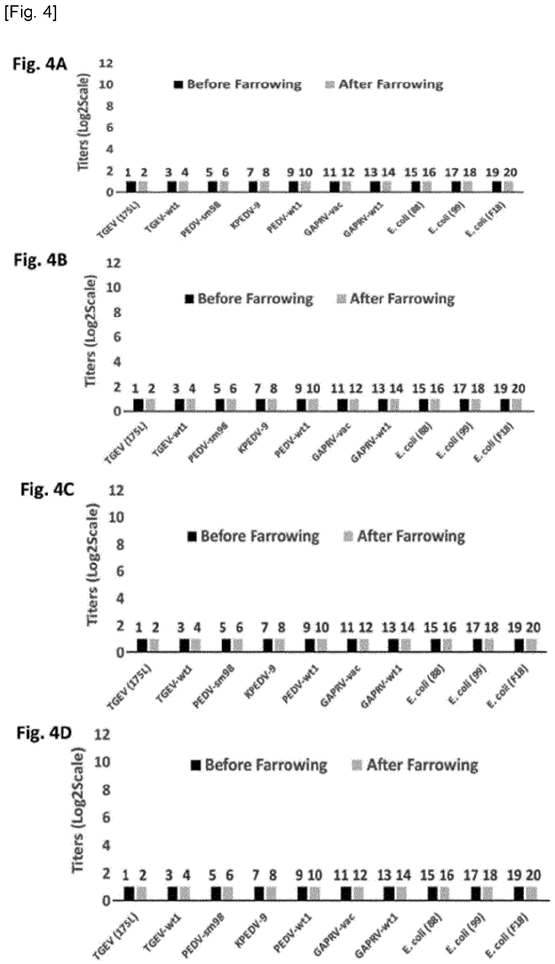

FIGs. 4A to 4D show the VN and AG titers against infectious pathogens in serums collected from the adult sows before and after farrowing. The serums were collected from four sows including sow 1, sow 2, sow 3, and sow 4 before and after farrowing. The VN titers of the collected serum against the TGEV(175L), the TGEV-wt1, the PEDV-sm98, the KPEDV-9, the PEDV-wt1, the GAPRV-vac and the GAPRV-wt1, and the AG titers of the collected serums against the E. coli(K88, K99, F18) were measured according to the materials and methods described herein, respectively. The number on each graph means a corresponding lane or column.
FIG. 4A shows the VN titers against the TGEV(175L), the TGEV-wt1, the PEDV-sm98, the KPEDV-9, the PEDV-wt1, the GAPRV-vac and the GAPRV-wt1, and the AG titers against the E. coli(K88, K99, F18) in the serum of sow 1 before and after farrowing.
FIG. 4B shows the VN titers against the TGEV(175L), the TGEV-wt1, the PEDV-sm98, the KPEDV-9, the PEDV-wt1, the GAPRV-vac and the GAPRV-wt1, and the AG titers against the E. coli(K88, K99, F18) in the serum of sow 2 before and after farrowing.
FIG. 4C shows the VN titers against the TGEV(175L), the TGEV-wt1, the PEDV-sm98, the KPEDV-9, the PEDV-wt1, the GAPRV-vac, and the GAPRV-wt1, and the AG titers against the E. coli(K88, K99, F18) in the serum of sow 3 before and after farrowing.
FIG. 4D shows the VN titers against the TGEV(175L), the TGEV-wt1, the PEDV-sm98, the KPEDV-9, the PEDV-wt1, the GAPRV-vac, and the GAPRV-wt1, and the AG titers against the E. coli(K88, K99, F18) in the serum of sow 4 before and after farrowing.

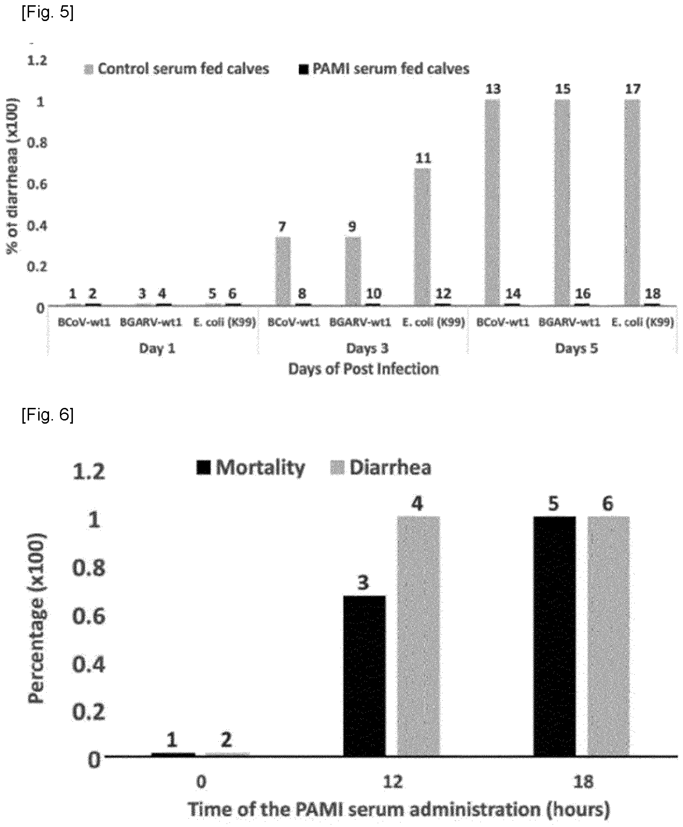

FIG. 5 shows clinical outcomes of control serum-fed or PAMI serum-fed calves after infection with the BCoV-wt1, the BGARV-wt1, and the E. coli(K99), respectively. In each of the calves' groups A, B and C, the control serum and the PAMI serum were administered to a subgroup "control serum-fed calves" and a subgroup "PAMI serum-fed calves," respectively. Then the calves' group A, B, and C were challenged with the BCoV-wt1, the BGARV-wt1, and the E. coli(K99), respectively, and clinical outcomes were examined for 5 days according to the materials and methods described herein. The number on each graph means a corresponding lane or column.
FIG. 6 shows a time of PAMI serum administration and clinical outcomes of the piglets challenged with the pathogen. The PAMI serums were orally administered to newborns at 0 hours after birth (n=3), at 12 hours after birth (n=3), and at 18 hours after birth (n=3), and the PAMI serum-fed piglets were challenged with the PEDV-wt1 (1×10⁶ TCID₅₀/a piglet) at three days old. Then, percentages of mortality and diarrhea incidence of the piglets were examined until 28 days after birth according to the materials and methods described herein. The number on each graph means a corresponding lane or column.

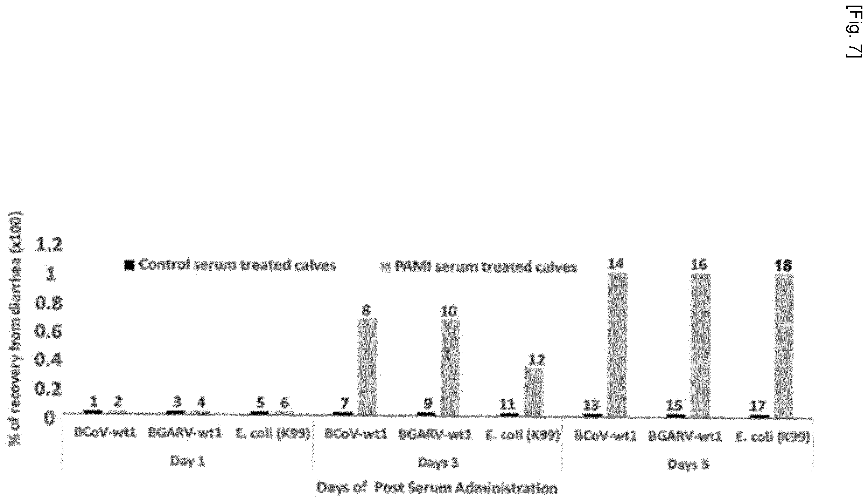

FIG. 7 shows recovery percentages of diarrheal calves infected with the BCoV-wt1, the BGARV-wt1, and the E. coli (K99) after the PAMI serum administration. The calves' group A [control serum-administered calves (n=3) and PAMI serum-administered calves (n=3)], the calves' group B [control serum-administered calves (n=3) and PAMI serum-administered calves (n=3)] and the calves' group C [control serum-administered calves (n=3) and PAMI serum-administered calves (n=3)] were infected with the BCoV-wt1, the BGARV-wt1, and the E. coli (K99), respectively. When the pathogen-infected calves show diarrhea, control serum and PAMI serum were administered to the subgroups, "control serum-administered calves" and "PAMI serum-administered calves," in each of the calves' groups, respectively, and clinical outcomes were examined according to the materials and methods described herein.

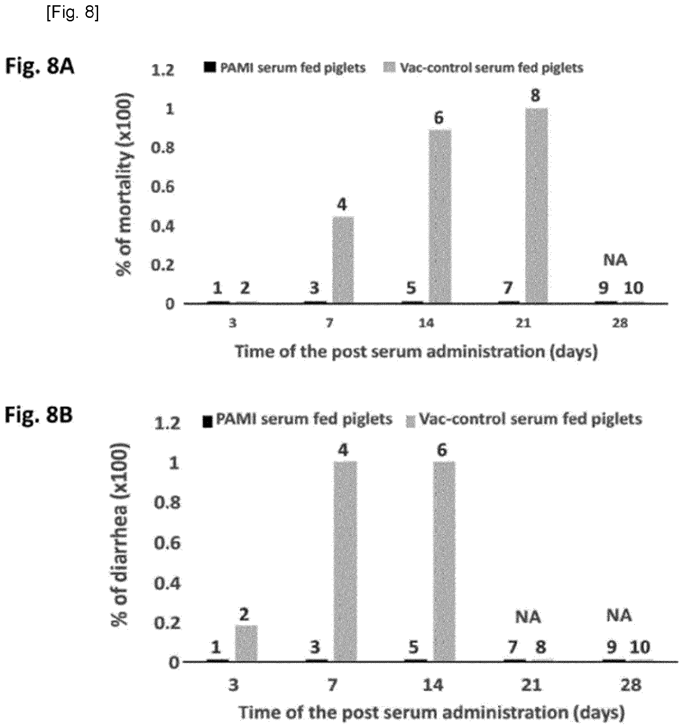

FIGs. 8A and 8B show clinical outcomes of the PAMI serum-fed piglets and the vac-control serum-fed piglets in a PEDV outbreak farm, respectively. The PAMI serums and the vac-control serums were orally administered to the PAMI serum-fed piglets' group (n=280) and the control serum-fed piglets' group (n=9), respectively, in the PEDV outbreak farm, and then clinical outcomes were examined until 28 days after birth according to the materials and methods described herein. The number on each graph means a corresponding lane or column. In FIGs. 8A and 8B, NA means not applicable.
FIG. 8A shows percentages of mortality in the PAMI serum-fed piglets and vac-control serum-fed piglets.
FIG. 8B shows percentages of diarrhea incidence in the PAMI serum-fed piglets and vac-control serum-fed piglets.

### MODES FOR THE INVENTION

As major etiological agents of the mucosa-related infectious diseases in the piglets and calves, the TGEV, the PEDV, the GAPRV, the BCoV, the BGARV, and the ETEC have caused contagious severe diarrhea with various morbidity and mortality, and the decreased growth performance, which lead to enormous economic losses to the pig and cattle livestock industry worldwide. However, an effective method for protecting young mammals against these mucosa-related infectious pathogens has not been developed.

As an example of solving such problems, the PAMI serum of the present invention, a serum containing protective antibodies with mucosal immunity against mucosa-related infectious pathogens including the TGEV, the PEDV, the GAPRV, and the ETEC, was induced and produced from an adult pig by orally administrating these mucosa-related infectious pathogens to the adult pig. When the PAMI serum was orally administered to the newborn piglets early after birth, the piglets were protected from challenge infection with the PEDV, the TGEV, the GAPRV, and the ETEC, respectively. In addition, the PAMI serum-fed piglets in the PEDV outbreak farm were completely protected from the PEDV infection and further spread of the PEDV successfully ceased in the PEDV outbreak farm.

Furthermore, to see whether the present invention, capable of protecting piglets from mucosa-related infectious pathogens, can be equally applied to other mammals with a similar immune system, another animal experiment was carried out in the calves. The PAMI serum capable of preventing mucosa-related infectious pathogens of calves was induced and produced from one adult cow by orally administering mucosa-related infectious pathogens, such as the BCoV, the BGARV, and the E. coli (K99), to an adult cow, and when the produced PAMI serum was orally administered to the neonatal calves, the PAMI serum-fed calves were successfully protected from the challenge with the BCoV-wt1, the BGARV-wt1, and the E. coli (K99), respectively. In addition, the diarrhea of the calves infected with the BCoV-wt1, the BGARV-wt1, and the E.coli (K99) was successfully treated by the intravenous administration of the PAMI serum.

As described above, the present invention was completed by experimentally confirming that the method of the present invention can be applied to the two different mammals and also to the various other mucosa-related infectious pathogens, and then the method of the present invention can prevent and/or treat infection with mucosa-related infectious pathogens in young mammals. These results strongly suggest that the method of the present invention can be applied to all mammals with a similar immune system, including ruminants, horses, and humans, as well as pigs and cattle as shown in the examples, for preventing and/or treating the infection with the mucosa-related infectious pathogens. In addition, the results suggest that the method of the present invention can be applied not only to the mucosa-related infectious pathogens of piglets and calves used in the examples but also to all of the mucosa-related infectious pathogens in young mammals.

In the present invention, the term "serum" means a material of plasma lacking fiber, and the PAMI serum contains protective antibodies with mucosal immunity that can protect young mammals from mucosa-related infectious pathogens.

In the method of producing a serum according to the present invention, mucosa-related infectious pathogens are a pathogenic wild type or an attenuated virus and bacteria that causes infectious diseases in digestive, respiratory, and reproductive organs. In addition, infection with these pathogens leads to mild clinical outcomes in adult pigs or adult cattle, but severe clinical outcomes in piglets or young calves.

In the method of producing a serum according to the present invention, the dose of the mucosa-related infectious pathogens is an amount that exhibits infection and immunogenicity but does not reach fatality in adult pigs and adult cattle, and that is sufficient to induce and produce the protective antibodies with mucosal immunity against infectious pathogens. In addition, a person skilled in the art can select and use an appropriate dose according to the type and condition of the mucosa-related infectious pathogen, and the frequency of administration can be adjusted so that an effective level of protective antibodies can be induced and generated in the serum.

In the method of producing a serum according to the present invention, the administration via mucosal route may be oral or nasal administration, and the protective antibodies with mucosal immunity against infectious pathogens can be created by administering the infectious pathogens via the oral or nasal route. Conventional vaccine compositions induce the production of antibodies by administering an immunogen via a non-mucosal route such as intramuscular injection, and since the serum containing protective antibodies induced and generated by antigen administration via the non-mucosal route lacks mucosal immunity, the conventional vaccines as an immunogenic source have a disadvantage that their effectiveness is not very high for preventing and/or treating infection with mucosa-related infectious pathogens.

In the method of producing a serum according to the present invention, the adult pig, or cow, means the adult mammal that has a fully developed immune system and can induce and generate protective antibodies with mucosal immunity when an infectious pathogen or its protein is administered. Preferably, the adult mammal may be a mammal including pigs, horses, ruminants, humans, and the like.

In the method of producing a serum according to the present invention, the PAMI serum capable of protecting against different mucosa-related infectious pathogens such as the TGEV, the PEDV, the GAPRV, the BCoV, the BGARV, and the E. coli, was induced and generated from one individual of pigs or cattle. Therefore, the PAMI serum, which can prevent and/or treat infection with multiple mucosa-related infectious pathogens, can be induced, produced, and maintained from one individual, a PAMI serum donor.

In addition, in the method of producing a serum according to an embodiment of the present invention, the protective antibodies include immunoglobulin specific to an infectious pathogen or its protein, and preferably, the protective antibodies contain mucosal immunity, include secretory IgA (slgA), which is secreted into the mucosa membrane as the main immunoglobulin of the protective antibodies, and can protect young mammals from mucosa-related infectious pathogens.

In addition, the present invention provides the PAMI serum that contains protective antibodies with mucosal immunity against infectious pathogens and can prevent and/or treat mucosa-related infectious diseases in young mammals.

In embodiments of the invention, the serum containing protective antibodies with mucosal immunity capable of protecting against infection with mucosa-related infectious pathogens was named "PAMI serum", the serum containing protective antibodies with mucosal immunity against mucosa-related infectious pathogens. The PAMI serum is characterized by its recognition of various epitopes of infectious pathogens or proteins thereof and in that protective antibodies with different antigen-recognition sites are mixed.

In addition, when the PAMI serum of the present invention is powdered through freeze-drying, the powdered PAMI serum can be stored for a long term, and the PAMI serum can be easily prepared through a simple step of adding a watersoluble solvent, such as water, before administering to a young mammal.

The serum containing the protective antibody with mucosal immunity capable of protecting against infection with the mucosal-related infectious pathogen according to the present invention can be used for passive immunity. The term "passive immunity" refers to immunity that an individual not receiving antigen stimulation acquires by injecting antibodies or sensitized lymphocytes of another individual, which are produced in an immune response by antigen stimulation via various routes. The passive immunity has an advantage that the recipient can obtain a protective effect in a short time without immunity to a pathogen compared to active immunity that induces the production of antibodies by vaccination.

In addition, the present invention provides a method of preventing and/or treating mucosa-related infectious diseases by administering an effective amount of serum containing protective antibodies with mucosal immunity capable of protecting against infection with mucosa-related infectious pathogens to newborn piglets, or young calves, via an oral or intravenous route.

In the method of preventing and/or treating mucosa-related infectious diseases according to the present invention, a PAMI serum recipient is a piglet, or a young calf, that cannot produce protective antibodies against infection with infectious pathogens because the immune system is not sufficiently developed or needs to acquire protective antibodies to defend against infection with infectious pathogens.

In the method of preventing and/or treating mucosa-related infectious diseases according to the present invention, the preferred administration of the serum containing the protective antibody with mucosal immunity capable of protecting against infection with the mucosa-related infectious pathogens may be oral or intravenous route but is not limited thereto.

In addition, the oral administration can be applied to newborn piglets, or calves, and used for preventing piglets, or young calves, from infection with mucosal-related infectious pathogens and preferably administered within 1 hour after birth of the newborn mammal. The time window for oral administration is a time window in which the serum of the present invention is administered to a newborn mammal, the protective antibodies capable of defending against infection with the mucosa-related infectious pathogens are absorbed, and the most remarkable passive immune effect exhibits.

In addition, the administration of the serum via intravenous route can be used for preventing and/or treating newborn and young piglets, or calves, from infection with mucosal-related infectious pathogens. The effective timing for intravenous administration in piglets, or calves, refers to a time when the administration of the serum containing the protective antibodies with mucosal immunity from the outside is necessary to prevent and/or treat infection with mucosa-related infectious pathogens.

The serum according to the present invention may be administered in an immunologically effective amount. The "immunologically effective amount" means an amount sufficient to exhibit the effect of preventing and/or treating infection with an infectious pathogen and an amount sufficient to not cause side effects or serious/excessive immune response. The precise administration concentration of the serum depends on the titer of the antibodies against a specific immunogen or infectious pathogen and can be easily determined by those skilled in the art according to factors well known in the medical field, such as the age, weight, health, sex, administration route, and administration method of the subject. Also, administration can be performed once to several times.

In addition, in the method of preventing and/or treating mucosa-related infectious diseases according to the present invention, the serum containing the protective antibodies with mucosal immunity capable of protecting against infection with the mucosa-related infectious pathogen may be administered to a newborn or young mammal along with one or more immune-enhancing agent.

The immune-enhancing agent that may be included in the present invention may refer to a substance that enhances immune response of a mammal to which the serum of the present invention is administered and may be many different adjuvants known to those skilled in the art. The immune-enhancing agent includes Freund's complete and incomplete immune-enhancing agent, vitamin E, Quil A, mineral oil, non-mineral oil, Carbopol, water-in-oil type emulsion adjuvant, and the like but is not limited thereto.

In the present invention, the results that the PAMI serum against mucosal-related infectious pathogens is induced and produced from two different mammals including pigs and cattle, and that the prevention and/or treatment of infection with mucosa-related infectious pathogens by using the PAMI serum were successful in piglets and calves indicate that the method of the present invention can be applied to horses, ruminants, primates, and others with a similar immune system.

As shown in examples of the present invention, it was confirmed that the method of the present invention can be applied to different mammals and various mucosa-related infectious pathogens for the prevention and/or treatment of infectious diseases in mammals. For this reason, pathogens to which the method of the present invention can be applied include not only the pathogens used in the examples, such as the TGEV, the PEDV, the GAPRV, the BCoV, the BGARV, the ETEC, but also all mucosa-related viral and bacterial infectious pathogens causing diseases in the digestive, respiratory, and genital organs of piglets and calves. In addition, pathogens to which the method of the present invention can be applied include all mucosa-related viral and bacterial infectious pathogens causing diseases in the digestive, respiratory, and reproductive organs of all mammals, including horses, ruminants, and primates with a similar immune system.

In addition, the infectious pathogens may be a virus or bacteria causing digestive or respiratory diseases in mammals and may include Rhinovirus, coronavirus, parainfluenza virus, respiratory syncytial virus, influenza virus, Adenovirus, norovirus, Enterovirus, Rotavirus, Calicivirus, Streptococcus pneumonia, Mycoplasma pneumonia, Hemophilus influenza, Klebsiella pneumonia, Chlamydia pneumoniaia, Vibrio cholera, Enterotoxigenic Escherichia coli, Salmonella spp., Clostridium spp., Shigella spp., Yelsinia spp., Porcine rotavirus, Transmissible gastroenteritis virus, Porcine epidemic diarrhea virus, Porcine parvovirus, Porcine reproductive and respiratory virus, Porcine respiratory coronavirus, Swine influenza virus, Swine vesicular disease virus, Porcine circovirus, Streptococcus Suis, Pasteurella multocida, Actinobacillus pleuropneumonia, Brachyspira hyodsenteriae, Bordetella brochiseptica, Hemophilus parasuis, Mycoplasma hyopneumoniae, Erysipelothrix rhusiopathiae, Malignant catarrhal fever virus, Bovine rotavirus, Bovine influenza virus, Bovine coronavirus, Bovine herpes virus, Bovine parainfluenza virus, Bovine respiratory syncytial virus, Bovine viral diarrhea virus, vesicular stomatitis virus, Bovine infectious rhinotracheitis virus, Bovine clostridium spp., Mannheimia haemolytica, African horse sickness virus, Equine virus influenza, Equine coronavirus, Streptococcus equi, Rhodococcus equi, and the like but is not limited thereto.

The present invention also provides a pharmaceutical composition for preventing and/or treating infectious diseases caused by infection with infectious pathogens, which comprises a serum containing protective antibodies with mucosal immunity capable of protecting against infection with the infectious pathogens, as an active ingredient.

The pharmaceutical composition of the present invention may further include a pharmaceutically acceptable carrier, excipient, or diluent in addition to the serum. In addition, the pharmaceutical composition may be formulated and used in the form of oral dosage forms such as suspensions, emulsions, syrups, and the like, and in the form of sterile injection solutions according to conventional methods but is not limited thereto. Carriers, excipients, and diluents that may be included in the composition may include lactose, dextrose, sucrose, sucrose, sorbitol, mannitol, xylitol, erythema, erythritol, starch, acacia rubber, alginate, gelatin, calcium phosphate, calcium silicate, cellulose, methylcellulose, tentative cellulose, water, and magnesium stearate. In the case of formulation, the pharmaceutical composition is prepared by using diluents or excipients such as commonly used fillers, extenders, binders, wetting agents, disintegrants, and surfactants. Liquid formulations for oral administration include suspensions, solutions, emulsions, syrups, and the like. In addition to water, which is a commonly used simple diluent, various excipients such as wetting agents, sweeteners, fragrances, and preservatives may be included. Formulations for parenteral administration include sterile aqueous solutions, nonaqueous solutions, suspensions, and emulsions.

A suitable dosage of the pharmaceutical composition according to the present invention can be determined in various ways depending on factors such as formulation, dosage, age, weight, sex, clinical condition, food, route of administration, antibody titer, the half-life of antibodies, response sensitivity, and the like.

The concentration of the active ingredient contained in the pharmaceutical composition of the present invention may be determined in consideration of the treatment purpose, the condition of the patient, the necessary period, and the like and is not limited to a concentration within a specific range.

The pharmaceutical composition of the present invention is not limited to the treatment of the infectious diseases described above and can be used for coronavirus infection, flu, an acute respiratory disease caused by influenza virus, adenovirus infection, parainfluenza virus infection, respiratory cell fusion Viral infection, rhinovirus infection, metapneumovirus infection, pneumococcal infection, mycoplasma infection, chlamydia infection, rotavirus infection, norovirus infection, enteroadenovirus infection, astrovirus infection, sapovirus infection, E. coli infection, Salmonella infection, and the like.

The present invention also provides a veterinary composition for preventing and/or treating infection with mucosal-related infectious pathogens in a neonatal or young mammal, which comprises a serum containing protective antibodies with mucosal immunity capable of protecting against infection with the infectious pathogens, as an active ingredient.

In the present invention, since the serum containing protective antibodies with mucosal immunity capable of protecting against infection with infectious pathogens has the effect of preventing and/or treating infectious diseases, the use of the PAMI serum can increase the immunity of livestock and improve and maintain the health of livestock.

The veterinary composition of the present invention may further include appropriate excipients and diluents commonly used in the production of veterinary compositions.

The veterinary dosage form of the serum according to the present invention may be used alone or in combination with other veterinary active compounds, as well as in any suitable group.

Excipients and diluents that may be included in the veterinary compositions comprising the serum of the present invention include lactose, dextrose, sucrose, sorbitol, mannitol, xylitol, erythritol, maltitol, starch, acacia rubber, alginate, gelatin, calcium phosphate, calcium silicate, cellulose, methylcellulose, cellulose, polyvinyl pyrrolidone, water, and magnesium stearate.

The veterinary composition comprising the serum according to the present invention may further include a filler, an anti-aggregator, a lubricant, a wetting agent, a spice, an emulsifier, a preservative, and the like, and be formulated using methods well known in the art to provide a rapid, sustained, or delayed release of an active ingredient after administration to an animal. Formulations of the composition may be in the form of suspensions, emulsions, solutions, syrups, sterile injectable solutions, and the like.

In veterinary compositions according to the present invention, the infectious pathogens may be a virus or bacteria that causes diseases in the digestive, respiratory, and genital organs of the mammals as described above.

In the method of the present invention, the PAMI serum capable of protecting against infections with mucosa-related infectious pathogens can be induced, maintained, and produced in an adult mammal by administering infectious pathogens to the adult mammal via the mucosal route, and administration of the PAMI serum via oral or intravenous route can prevent and/or treat infection with the mucosa-related infectious pathogens in young mammals. Therefore, when the method of the present invention is applied to all mammals with a similar immune system and mucosa-related infectious pathogens, it can be a new method to prevent and/or treat infection with mucosa-related infectious pathogens that cannot be solved by existing methods, such as vaccines, or to replace the existing methods in controlling infectious diseases of young mammals.

Hereinafter, the present invention will be described in detail with examples. However, the following examples are only illustrative of the present invention, and the present invention is not limited to the following examples.

### Example 1. Materials and Methods

### 1-1. Cells, Viruses, and Bacteria

Vero cells, swine testis (ST) cells, and Fetal rhesus monkey kidney (MA104) cells were cultured at 37°C in a CO₂ incubator, by using the eagle's minimum essential medium (EMEM) or Dulbecco's Modified Eagle's Medium (DMEM) containing 5-10% fetal bovine serum (FBS), penicillin (100 units/ml), and streptomycin (100 µg/ml). Wild type porcine epidemic diarrhea virus (PEDV-wt1), wild type transmissible gastroenteritis virus (TGEV-wt1), wild type group A porcine rotavirus (GAPRV-wt1), wild type bovine coronavirus (BCoV-wt1), wild type bovine group A rotavirus (BGARV-wt1), commercial vaccine virus strains [KPEDV-9 (Korea BNP, Korea), PEDV-m98 (Green Cross Veterinary Products Co., LTD, Korea), TGEV(175L) (Green Cross Veterinary Products Co., LTD, Korea), GAPRV-vac (Green Cross Veterinary Products Co., LTD, Korea), BGARV-vac (Korea, BNP)], and the E. coli (K88, K99 and F18, kindly obtained from Veterinary Science Research Institute, Korea) were used for the experiments. The PEDV and the BCoV were propagated in the Vero cells by using DMEM containing 10µg/ml trypsin, the TGEV was propagated in the ST cells by using the DMEM, and the GAPRV and the BGARV were propagated in the MA104 cells by using the EMEM (or the DMEM) containing 0.5-1.5 µg/ml trypsin, respectively. The E. coli were cultured in LB broth (Affymetrix, Inc. OH, USA).

### 1-2. Isolation of Wild Type Viruses

Wild type TGEV (Zhenhui S et al., 2015. Israel Journal of Veterinary Medicine 70:22-30), wild type PEDV and wild type BCoV (Chen Q et al., 2014. J Clin Microbiol 52:234-243), and wild type GAPRV and wild type BGARV (Arnold M et al., 2009. Curr Protoc Microbiol Chapter 15:Unit 15C 13) were isolated from feces of the piglet or calves showing the typical clinical signs to each virus infection according to the described in the published methods and were named the TGEV-wt1, the PEDV-wt1, the BCoV-wt1, the GAPRV-wt1, and the BGARV-wt1, respectively.

### 1-3.Titration of Viruses

Viral titration assays were performed according to the previously described method. For titration of the PEDV and the BCoV (Thomas JT et al., 2015. PLoS One 10:e0139266), confluent 80-90% monolayer Vero cells on the 96-well culture plate were prepared and then PEDVs or BCoVs activated by the trypsin with a final concentration of 10 µl/ml at 37°C for 1 hour were serially diluted. 0.1 ml of the diluted PEDV, or the BCoV, was inoculated into each well of Vero cells, which were washed with PBS three times. After adsorption for 1 hour at 37°C, the Vero cells with inoculums were replaced with 0.2 ml of the DMEM containing trypsin (10 µg/ml) without the FBS, and the Vero cells were further incubated for 3-5 days at 37°C in a CO₂ incubator. For the TGEV titration (He L et al., 2012. Virol J 9:176), confluent ST cells on a 96-well culture plate were washed with PBS three times, and 0.1 ml of serially diluted viruses was added into each well of the ST-cell culture plate. After 1 hour of absorption at 37°C, the ST cells with inoculums were replaced with 0.2 ml of the DMEM containing 5% FBS followed by incubation at 37°C for 3-5 days in a CO₂ incubator. For GAPRV and BGARV titration (Otto PH et al., 2015. J Virol Methods 223:88-95), confluent 80-90% monolayer MA104 cells on the 96-well cultured plate were washed with the PBS three times, and 0.1ml of serially diluted GAPRVs, or BGARVs, that were activated by trypsin with a final concentration of 10 µg/ml at 37°C for 1 hour was inoculated into each well with MA104 cells. After 1 hour of attachment at 37°C, the MA104 cells with inoculums were replaced with 0.2 ml of the EMEM, or DMEM, containing 0.5-1.5 µg/ml trypsin without the FBS and then further incubated at 37°C for 5-7 days in a CO₂ incubator. When the cytopathic effect (CPE) was observed, the virus inoculated cells were fixed with 10% formalin for 30 min at room temperature followed by staining with 0.5% crystal violet (w/v). Median tissue culture infectious doses (TCID₅₀) were expressed as a reciprocal of the highest virus dilution number showing plaques by cytopathic effect (CPE) for each virus.

### 1-4. Animals and Inoculation of Infectious Pathogens

Five pregnant sows were purchased from a pig farm and raised in separate rooms, respectively. Four three months old pigs including two female and two castrated male pigs were purchased from the pig farm and raised in the same place. For the production of the serum containing protective antibodies with mucosal immunity against mucosa-related infectious pathogens of the piglets, named the PAMI serum, each 40 ml of 10⁶ TCID₅₀ viruses [TGEV(175L), TGEV-wt1, PEDV-m98, KPEDV-9, PEDV-wt1, GAPRV-vac and GAPRV-wt1], and 300 ml of overnight culture E. coli (K88, K99 and F18) were mixed with the feed and then the mixture was orally fed into two male pigs and two female pigs at every week for 26 weeks. For the production of the vaccinated control serum, named the vac-control serum, one adult pig was intramuscularly vaccinated with commercial vaccines (KPEDV-9 and PEDV-m98) according to the manufacturer's directions. Then, additional two doses of the vaccines were given at intervals of 2 weeks.

For the production of the serum containing protective antibodies with mucosal immunity against mucosa-related infectious pathogens of the calves, named the PAMI serum, 15 months old female cow was used. The 40 mℓ of each virus (1×10⁶ TCID₅₀/mℓ) [BCoV-vac, BCoV-wt1, BGARV-vac, and BGARV-wt1] and 100 mℓ of overnight cultured E. coli (K99) were orally fed into the female cow every week for 12 weeks.

### 1-5. Serum and Titration

The serums were collected from four sows before and after farrowing. The control serums were collected from four adult pigs before challenge with the infectious pathogens such as the PEDV, the TGEV, the GAPRV, and the E. coli, the PAMI serums were collected from the four adult pigs at 26 weeks after challenge with the infectious pathogens, and control serum and vac-control serum were collected from the pig before and after vaccination with the KPEDV-9 and the PEDV-sm98, respectively. Each serum was collected through a milk vein or a carotid artery, respectively. In addition, the serum of the adult cows before (weeks 0) and after (weeks 12) infection with the BCoV-vac, the BCoV-wt1, the BGARV-vac, the BGARV-wt1, and the E. coli (K99) were collected through a jugular vein, respectively. The collected serums of the pigs were all examined for VN titers against the PEDV, the TGEV, and the GAPRV and AG titers against the E. coli. In addition, the collected serums of the cows were all examined for VN titers against the BCoV-vac, the BCoV-wt1, the BGARV-vac, and the BGARV-wt1 and AG titers against the E. coli (K99).

The VN titers of the serum against the TGEV, the PEDV, the GAPRV, the BCoV, and the BGARV were examined according to previously published methods (Arnold M et al, 2009. Curr Protoc Microbiol Chapter 15:Unit 15C 13; Woods RD et al., 1988. Am J Vet Res 49:300-304; Song DS et al., 2007. Res Vet Sci 82:134-140). Briefly, the serums were heated at 56°C for 30 minutes, and doubling dilutions of each serum with the DMEM, or EMEM, were performed in the round-bottomed micro-titer 96 well plates. The 50µl of the activated virus (200 TCID₅₀/50µl) and the 50µl of the diluted serum were mixed and incubated for 1 hour at 37°C. The 100 µl of PEDV/serum mixture, or BCoV/serum mixture, was added into monolayer Vero cells on the 96-well culture plate; the 100 µl of TGEV/serum mixture was added into monolayer ST cells on the 96-well culture plate; and the 100 µl of GAPRV/serum mixture, or BGARV/serum mixture, was added into monolayer MA104 cells on the 96-well culture plate, respectively. Then, the mixtures further were incubated for 2 hours at 37°C. The inoculums of the virus/serum mixture were replaced with 0.2 ml of the DMEM containing 5% FBS for the TGEV/ serum mixture, 0.2 ml of the DMEM containing 10µg/ml trypsin for the PEDV/serum mixture, or the BCoV/serum mixture, and 0.2 ml of the EMEM, or the DMEM, containing 0.5-1.5µg/ml trypsin for the GAPRV/serum mixture, or the BGARV/serum mixture, respectively. The virus-inoculated cell plates were incubated for 3-7 days at 37°C in a CO₂ incubator. When the virus inoculated cells distinctly showed CPE, the cells were fixed with 10% formalin in the PBS (200µl/well) for 30 min at room temperature followed by staining with 0.5% crystal violet (w/v). Then, the VN titer was expressed as a reciprocal of the highest dilution number of the antiserum that did not show the CPE on the monolayer cells with the virus/serum mixture.

To determine the serum titers to the E. coli, AG assays were performed as described. The E. coli was grown in the LB broth to an estimated 5 × 10⁹ cells/ml followed by 1% formalin treatment (vol/vol) for 12 hours. Formalized cultures were washed 3 times with the PBS, resuspended in the PBS at the OD₆₀₀ of 0.7, and used as an antigen for serum titration. 100 µl of the E.coli antigen was mixed with 100 µl of serially diluted antiserum in a 96 U-shaped plate followed by incubation at 37°C for 3 hours. The agglutination titer of the serum was expressed as a reciprocal of the highest dilution of the test sample exhibiting distinct agglutination of the test antigen.

### 1-6. Animal Experiments

To examine whether the PAMI serums from the adult pig can prevent infectious diseases of the piglets, pathogen challenge experiments were performed in the piglets from four sows. The piglets from each of the four sows were designated as the piglets' group A [control serum-fed piglets (n=7) and PAMI serum-fed piglets (n=3)] for the TGEV-wt1 challenge, the piglets' group B [control serum-fed piglets (n=8) and PAMI serum-fed piglets (n=3)] for the PEDV-wt1 challenge, the piglets' group C [control serum-fed piglets (n=7) and PAMI serum-fed piglets (n=3)] for the GAPRV-wt1 challenge, and the piglets' group D [control serum-fed piglets (n=8) and PAMI serum-fed piglets (n=3)] for the E. coli (K88) challenge, respectively. Piglets of each group were further divided into the PAMI serum-fed piglets and control serum-fed piglets, respectively, as mentioned above. 10 ml of the PAMI serum and 10 ml of the control serum were orally administered into the PAMI serum-fed piglets and control serum-fed piglets within 1 hour after birth, respectively. Two days after birth, all the piglets were separated from the sows, raised in a different place and fed with the milk replacer (Purina, Korea) until the end of the experiment. At three days after birth, the piglets' groups A, B, C, and D were orally challenged with the 1×10⁶TCID₅₀ TGEV-wt1/a piglet, the 1×10⁶ TCID₅₀ PEDV-wt1/a piglet, the 1×10⁶TCID₅₀ GAPRV-wt1/a piglet, and the 1×10⁸ CFU E. coli (K88)/a piglet, respectively, and clinical outcomes of the piglets were observed for four weeks.

To examine whether the protection of the piglets from the infectious pathogens is affected by the time of administering the PAMI serum, pathogen challenge experiments were performed on the piglets' group E [subgroup 0 hours (n=3), subgroup 12 hours (n=3), and subgroup 18 hours (n=3)]. Each of the three subgroups from one litter was orally fed with 10 ml of the PAMI serum at different time points of 0 hours, 12 hours, and 18 hours after birth, respectively. Three days after birth, all three groups of the PAMI serum-fed piglets were challenged with the 1×10⁶ TCID₅₀ PEDV-wt1/a piglet and raised with the mother sow, and then clinical outcomes of PEDV infected piglets were examined.

To examine whether the PAMI serum from the cattle can prevent the mucosa-related infectious diseases of the calves, pathogen challenge experiments were performed on the calves. Calves were divided into the calves' group A [control serum-fed calves (n=3) and PAMI serum-fed calves (n=3)] for the BCoV-wt1 challenge, the calves' group B [control serum-fed calves (n=3) and PAMI serum-fed calves (n=3)] for the BGARV-wt1 challenge, and the calves' group C [control serum-fed calves (n=3) and PAMI serum-fed calves (n=3)] for the E. coli (K99) challenge, respectively. 200ml of the PAMI serum and 200 ml of the control serum were orally administered to the PAMI serum-fed calves and control serum-fed calves within 1 hour after birth, respectively. At three days after administration of the PAMI and control serums, the calves' groups A, B, and C were orally challenged with the BCoV-wt1(1×10⁶ TCID₅₀/a calf), the BGARV-wt1(1×10⁶ TCID₅₀/a calf), and the E. coli (K88) (1×10⁸ CFU/a calf), respectively, and clinical outcomes of the calves challenged with infectious pathogens were examined for 5 days.

To examine whether diarrhea of the calves infected with the BoCoV-wt1, the BGARV-wt1, or the E. coli (K99) can be treated by intravenous administration of the PAMI serum, animal experiments were carried out in calves. Each of the calves' groups was further divided into control serum-administered calves and PAMI serum-administered calves, respectively. Then, each of the calves' groups D, E, and F was challenged with the BCoV-wt1(1×10⁶ TCID₅₀/a calf) [control serum-administered calves (n=3) and PAMI serum-administered calves (n=3)], the BGARV-wt1 (1×10⁶ TCID₅₀/a calf) [control serum administered calves (n=3) and PAMI serum-administered calves (n=3)], and the E. coli(K88) (1×10⁸ CFU/a calf) [control serum-administered calves (n=3) and PAMI serum-administered calves (n=3)], respectively. At three days after challenge with the infectious pathogens, the PAMI serum and control serum were intravenously administered to PAMI serum-administered calves and control serum-administered calves that show the sign of diarrhea, respectively, and then clinical outcomes were examined for five days.

### 1-7. Use of PAMI Serum in PEDV Outbreak Farm

To examine whether the PAMI serums can prevent infectious diseases in the field, the PAMI serums were applied to the newborn piglets in the PEDV outbreak farm (200 sow units). 10 ml of the PAMI serum and 10 ml of the vac-control serum were orally administered into the PAMI serum-fed piglets' group (n=280) and the vac-control serum-fed piglets' group (n=9) within 1 hour after birth, respectively, and the clinical outcomes of the piglets were observed for 4 weeks.

### Example 2. VN and AG Titers of PAMI Serums against Mucosa-related Infectious Pathogens

A neutralizing ability of antibodies in a serum is one of the important aspects to determine a protective ability of the serum against infectious pathogens. Thus, the serums from the adult pigs infected with the PEDV, the TGEV, the GAPRV, and the E. coli, and from cattle infected with the BCoV, the BGARV, and the E. coli (K99) were evaluated by VN and AG assays, respectively.

In the serum of adult pigs, the control serum of adult male and female pigs before infection showed negative VN titers to the TGEV (L75), the TGEV-wt1, the PEDV-sm98, the KPEDV-9, the PEDV-wt1, the GAPRV-vac, and the GAPRV-wt1 (FIGs. 1A and 1B, lanes 1, 3, 5, 7, 9, 11, and 13), and negative AG titers to the E. coli (K88, K99 and F18) (FIGs. 1A and 1B, lanes 15, 17, and 19), respectively. However, the PAMI serum of the adult male and female pigs collected at 26 weeks after infection showed over 1024 VN titers to the TGEV(175L), the TGEV-wt1, the PEDV-sm98, the KPEDV-9, the PEDV-wt1, the GAPRV-vac, and the GAPRV-wt1 (FIGs. 1A and 1B, lanes 2, 4, 6, 8, 10, 12, and 14), and over 64 AG titers to the E. coli (K88, K99 and F18) (FIGs. 1A and 1B, lanes 16, 18, and 20), respectively. In addition, the vac-control serum of the pigs vaccinated with the PEDV-sm98 and the KPEDV-9 vaccines showed over 1024 VN titers against the PEDV-sm98, the KPEDV-9, and the PEDV-wt1 (FIGs. 1C, lanes 2, 4, and 6). However, the unvaccinated pig serum, which is the control serum, showed the negative VN titers to the PEDV-sm98, the KPEDV-9, and the PEDV-wt1 (FIG. 1C, lanes 1, 3, and 5), respectively.

In the serum of the adult cow, the control serum of the cow before infection showed negative VN titers to the BCoV-wt1, the BCoV-vac, the BGARV-wt1, and the BGARV-vac (FIG. 2, lanes 1, 3, 5, and 7), and negative AG titers to E. coli (K99) (FIG. 2, lane 9), respectively. However, the PAMI serum of the cow at 12 weeks after infection showed over 256 VN titers against the BCoV-wt1, the BCoV-vac, the BGARV-wt1, and the BGARV-vac (FIG. 2, lanes 2, 4, 6, and 8) and over 64 AG titer to the E. coli(K99) (FIG. 2, lane 10), respectively.

In general, the titer of the serum against infectious pathogens is one way to measure the protective ability against infection with infectious pathogens. Therefore, the above experimental results that the PAMI serums from the pigs and cow show high VN and AG titers against infectious pathogens suggest a possibility that the piglets and calves can be protected from infection with the mucosa-related infectious pathogens by acquiring protective antibodies of the PAMI serums.

### Example 3. Protective Effect of PAMI Serums in Piglets and Calves Challenged with Infectious Pathogens

To examine whether the PAMI serum of the adult pigs can protect the piglets from challenge with the PEDV-wt1, the TGEV-wt1, the GAPRV-wt, and the E. coli(K88), and whether the PAMI serum of the adult cows can protect the calves from challenge with the BCoV-wt1, the BGARV-wt1, and the E. coli(K88), pathogen challenge experiments were performed in the piglets and calves, respectively.

For the piglets' groups A, B, C, and D, the PAMI serum and control serum were orally administered to the "PAMI serum-fed piglets" and "control serum-fed piglets," respectively. At three days after serum administration, the piglets' groups A, B, C, and D were challenged with the PEDV-wt1, the TGEV-wt1, the GAPRV-wt1, and the E. coli(K88), respectively, and clinical outcomes were examined for 28 days.

In the piglets' group A challenged with the TGEV-wt1, the PAMI serum-fed piglets showed 0% mortality (FIG. 3A, lane 1) and 33% diarrhea incidence (FIG. 3B, lane 1), and the control serum-fed piglets showed 100% mortality (FIG. 3A, lane 2) and 100% diarrhea incidence (FIG. 3B, lane 2), respectively. In the piglets' group B challenged with the PEDV-wt1, the PAMI serum-fed piglets showed 0% mortality (FIG. 3A, lane 3) and 33% diarrhea incidence (FIG. 3B, lane 3), and the control serum-fed piglets showed 100% mortality (FIG, 3A, lane 4) and 100% diarrhea incidence (FIG. 3B, lane 4), respectively. In the piglets' group C challenged with the GAPRV-wt1, the PAMI serum-fed piglets showed 0% mortality (FIG. 3A, lane 5) and 0% diarrhea incidence (FIG. 3A, lane 5), and the control serum-fed piglets showed 29% mortality (FIG. 3A, lane 6) and 100% diarrhea incidence (FIG. 3B, lane 6), respectively. In the piglets' group D challenged with the E.coli(K88), the PAMI serum-fed piglets showed 0% mortality (FIG. 3A, lane 7) and 33% diarrhea incidence (FIG. 3B, lane 7), and the control serum-fed piglets showed 25% mortality (FIG. 3A, lane 8) and 100% diarrhea incidence (FIG. 3B, lane 8), respectively.

However, there is a possibility that the protection of the PAMI serum-fed piglets from the PEDV-wt1, the TGEV-wt1, the GAPRV-wt1, and the E. coli(K88) may be due to the colostrum containing protective antibodies of sows, which were infected with the PEDV, the TGEV, the GAPRV, and the E.coli before farrowing,-and have the protective antibodies against these infectious pathogens. To exclude this possibility, VN and AG titers of the serums of the sows before and after farrowing were examined. The serums of the sows before and after farrowing showed negative VN titers against the TGEV(175L), the TGEV-wt1, the PEDV-sm98, the KPEDV-9, the PEDV-wt1, the GAPRV-vac, and the GAPRV-wt1 (FIGs. 4A to 4D, lanes 1-14) and negative AG titers against the E. coli (K88), the E. coli(K99), and the E. coli(F18) (FIGs. 4A to 4D, lanes 15-20), respectively.

In addition to the piglets, to examine whether the method of the present invention can be applied to different species of mammals, the PAMI serum against the BCoV-wt1, the BCoV-vac, the BGARV-wt1, the BGARV-vac, and the E. coli(K99) was produced from the cow and pathogen challenge experiments were carried out in the calves. In the calves' groups A, B, and C, the PAMI serum and the control serum were administered to the PAMI serum-fed calves and the control serum-fed calves, respectively. At 3 days after serum administration, the calves' groups A, B, and C were challenged with the BCoV, the BGARV, and the E. coli (K99), respectively, and clinical outcomes were examined for five days. In the calves' group A challenged with the BCoV-wt1, control serum-fed calves showed 0%, 33% and 100% diarrhea incidence at day 1, day 3 and day 5 after the virus challenge (FIG. 5, lanes 1, 7, and 13), respectively, and PAMI serum-fed calves showed 0% diarrhea incidence at day 1, day 3 and day 5 after the virus challenge (FIG. 5, lanes 2, 8, and 14). In the calves' group B challenged with the BGARV-wt1, control serum-fed calves showed 0%, 33% and 100% diarrhea incidence at day 1, day 3 and day 5 after the virus challenge (FIG. 5, lanes 3, 9, and 15), respectively, and PAMI serum-fed calves showed 0% diarrhea incidence at day 1, day 3, and day 5 after virus challenge (FIG. 5, lanes 4, 10, and 16). In the calves' group C challenged with the E. coli (K99), control serum-fed calves showed 0%, 37% and 100% diarrhea incidence at day 1, day 3 and day 5 after virus challenge (FIG. 5, lanes 5, 11, and 17), respectively, and PAMI serum-fed calves showed 0% diarrhea incidence at day 1, day 3 and day 5 after the virus challenge (FIG. 5, lanes 6, 12, and 18).

The results of the pathogen challenge experiments indicate that the PAMI serum can be induced and produced from an adult pig or adult cattle; the PAMI serum can be passively transferred to the piglets or calves via the oral route; and the PAMI serum-administered piglets or calves can be protected from the infection with the mucosa-related infectious pathogens.

### Example 4. Time of PAMI Serum Administration and Protection of Piglets from Infectious Pathogens

In mammals such as horses, pigs, and ruminants that acquire passive immunity via colostrum, the timing of the colostrum intake is an important factor that affects a degree of passive immunity of offspring. Therefore, the animal experiment was conducted in the piglets to examine the relationship between the timing of oral administration of PAMI serum to newborn mammals and the protection of PAMI serum-administered young mammals from the infection with infectious pathogens.

In the piglets' group E, the PAMI serums were orally administered to the newborn piglets at 1 hour, 12 hours, and 18 hours after birth, respectively. Then, the PAMI serum-fed piglets were challenged with the PEDV-wt1 at 3 days after birth, and clinical outcomes were examined for 28 days. The PAMI serum-administered piglets within one hour after birth did not show any clinical symptoms such as mortality and diarrhea (FIG. 6, lanes 1 and 2), the PAMI serum-administered piglets within 12 hours after birth showed 66% mortality and 100% diarrhea incidence (FIG. 6, lanes 3 and 4), and the PAMI serum-administered piglets within 18 hours after birth showed 100% mortality and 100% diarrhea incidence (FIG. 6, lanes 5 and 6), respectively.

The results indicate that early oral administration of the PAMI serum to the piglets can effectively protect the piglets from the infection with infectious pathogens and suggest that this method of the present invention can be applied to the mammals such as pigs, horses, and ruminants in which maternal antibodies are transferred into the offspring via colostrum.

### Example 5. Therapeutic Effect of PAMI Serum on Diarrhea of Calves Infected with BCoV-wt1, BGARV-wt1, or E. coli (K99)

To examine whether the protection of piglets and/or calves from infection with mucosa-related infectious pathogens can be achieved by intravenous administration of PAMI serum as well as by an oral administration, experiments were conducted on calves. The PAMI serum was intravenously administered into diarrheal calves infected with the BCoV-wt1, the BGARV-wt1, or the E. coli (K99), respectively, and the clinical outcomes of the calves were examined for 5 days.

In the calves' group D infected with the BCoV-wt1, control serum-administered calves showed 0% recovery at day 1, day 3, and day 5 after serum administration (FIG. 7, lanes 1, 7, and 13), and PAMI serum-administered calves showed 0%, 67% and 100% recovery at day 1, day 3, and day 5 after serum administration (FIG. 7, lanes 2, 8, and 14), respectively. In the calves' group E infected with the BGARV-wt1, control serum-administered calves showed 0% recovery at day 1, day 3, and day 5 after serum administration (FIG. 7, lanes 3, 9, and 15), and PAMI serum-administered calves showed 0%, 67% and 100% recovery at day 1, day 3, and day 5 after serum administration (FIG. 7, lanes 4, 10, and 16), respectively. In the calves' group F infected with the E. coli (K99), control serum-administered calves showed 0% recovery at day 1, day 3 and day 5 after serum administration (FIG. 7, lanes 5, 11, and 17), and PAMI serum-administered calves showed 0%, 33% and 100% recovery at day 1, day 3, and day 5 after serum administration (FIG. 7, lanes 6, 12, and 18), respectively.

Unlike oral administration, which is affected by the neonatal gut closure and administration time of the PAMI serum, intravenous administration of the protective antibodies of the PAMI serum to young mammals can be effectively applied when necessary. Therefore, the results indicate that the method of the present invention can be used for treating as well as preventing infection with infectious pathogens in young mammals.

### Example 6. Use of PAMI serum in PEDV Outbreak Farm

To examine whether the PAMI serum of the present invention can effectively protect young mammals from infectious pathogens even in the field, the PAMI serum and the vac-control serum were orally administered to the PAMI serum-fed piglets (n=280) and the control serum-fed piglets (n=9), respectively, in the PEDV outbreak farm (200 sow units). Then clinical outcomes of the piglets were observed for 28 days after birth, and the effect of the PAMI serum was evaluated. The PAMI serum-fed piglets did not show any mortality (FIG. 8A, lanes 1, 3, 5, 7, and 9) and diarrhea incidence (FIG. 8B, lanes 1, 3, 5, 7, and 9) until 28 days old. However, the vac-control serum-fed piglets, which were administered with the vac-control serum from intramuscularly vaccinated adult pigs, showed 44%, 89%, and 100% mortality at 7, 14, and 21 days old (FIG. 8A, lanes 2, 4, and 6), respectively. In addition, the vac-control serum-fed piglets showed 33% diarrhea incidence at 3 days old (FIG. 8B, lane 2), 100% diarrhea incidence at 7 days old (FIG. 8B, lane 4), and 100% diarrhea incidence at 14 days old (FIG. 8B, lane 6), respectively.

These results indicate that the method of the present invention can effectively protect young mammals from infection with infectious pathogens not only in pathogen challenge experiments but also in PEDV outbreak farms and that PAMI serum containing the protective antibodies capable of protecting infection with mucosa-related infectious pathogens can be induced, produced, and maintained in an adult mammal regardless of the gender of the PAMI serum donor. In addition, the PAMI serum capable of protecting against infection with mucosa-related infectious pathogens can be induced and produced when infectious pathogens are administered into a PAMI serum donor via a mucosal route such as an oral or nasal route, not a non-mucosal route such as a subcutaneous, intradermal, or intramuscular route.

As shown in examples according to the method of the present invention, different species of young mammals were prevented and/or treated from infection with a variety of different mucosa-related infectious pathogens. These results suggest that the method of the present invention for preventing and/or treating the infection with infectious pathogens can be applied not only to pigs and cattle used in the examples, but also to all mammals with a similar immune system to the pigs and cattle, and then it could be applied not only to mucosa-related infectious pathogens used in the examples, but also to all mucosa-related infectious pathogens that cause infectious diseases in the digestive, respiratory, and reproductive organs of young mammals.

## Claims

1. A method of producing a serum containing a protective antibody with mucosal immunity capable of protecting against infection with a mucosa-related infectious pathogen, the method comprising a step of:
inducing production of the protective antibody with mucosal immunity capable of protecting against infection with the mucosa-related infectious pathogen, from an adult mammal, by administering the mucosa-related infectious pathogen to the adult mammal through an oral or nasal route.

2. The method of producing a serum of claim 1, wherein the mammal is a pig, a horse, a ruminant, or human.

3. The method of producing a serum of claim 1, wherein the mucosa-related infectious pathogen is a mucosa-related infectious viral pathogen or a mucosa-related infectious bacterial pathogen that causes a disease in a digestive organ, a respiratory organ, or a genital organ of a young mammal.

4. The method of producing a serum of claim 3, wherein the mucosa-related infectious viral pathogen or the mucosa-related infectious bacterial pathogen that causes a disease in a digestive organ, a respiratory organ, or a genital organ of a young mammal is any one of coronavirus, rotavirus, or enterotoxigenic Escherichia coli.

5. The method of producing a serum of claim 1, wherein the protective antibody with mucosal immunity includes secretory immunoglobulin A (slgA) as a primary antibody, which protects against infection with the mucosa-related infectious pathogen by being secreted to a mucous membrane.

6. A serum produced by the method of any one of claims 1 to 5, wherein the serum contains a protective antibody with mucosal immunity capable of protecting against infection with a mucosa-related infectious pathogen.

7. A method of preventing or treating infection with a mucosa-related infectious pathogen in a neonatal or young mammal comprising a step of:
administering an effective amount of the serum of claim 6 containing the protective antibody with mucosal immunity capable of protecting against infection with the mucosa-related infectious pathogen.

8. The method of preventing or treating infection with a mucosa-related infectious pathogen of claim 7, wherein the step of administering the serum is performed by administering the serum into the neonatal mammal within 1 hour after birth through an oral route in order to prevent infection with the mucosa-related infectious pathogen and by administering the serum into the young mammal through a vein in order to prevent and treat infection with the mucosa-related infectious pathogen.

9. A veterinary composition for preventing or treating infection with a mucosa-related infectious pathogen in a neonatal or young mammal, the veterinary composition comprising the serum of claim 6 as an active ingredient,
wherein the serum contains the protective antibody with mucosal immunity capable of protecting against infection with the mucosa-related infectious pathogen.

10. A pharmaceutical composition for preventing or treating a disease caused by infection with an infectious pathogen, the pharmaceutical composition comprising the serum of claim 6 as an active ingredient,
wherein the serum contains the protective antibody with mucosal immunity capable of protecting against infection with the mucosa-related infectious pathogen.
